(19) Europäisches Patentamt
European Patent Office
Office européen des brevets

(11) Veröffentlichungsnummer: **0 549 927 A2**

# (12) EUROPÄISCHE PATENTANMELDUNG

(21) Anmeldenummer: **92120946.6**

(22) Anmeldetag: **09.12.92**

(51) Int. Cl.[5]: **C07D 413/12**, //(C07D413/12, 263:00,207:00)

(30) Priorität: **20.12.91 DE 4142188**

(43) Veröffentlichungstag der Anmeldung: **07.07.93 Patentblatt 93/27**

(84) Benannte Vertragsstaaten: **AT BE CH DE DK ES FR GB GR IT LI LU MC NL PT SE**

(71) Anmelder: **BAYER AG**

**W-5090 Leverkusen 1 Bayerwerk(DE)**

(72) Erfinder: **Hoppe, Dieter, Professor Dr.**
**Schneiderkamp 17e**
**W-2300 Kiel(DE)**
Erfinder: **Schwerdtfeger, Jörg**
**Lensahner Strasse 13**
**W-2300 Kiel 14(DE)**

## (54) Heterocyclisch substituierte Carbamate und Verfahren zu ihrer Herstellung.

(57) Die Erfindung betrifft neue heterocyclisch substituierte Carbamate der Formel (I)

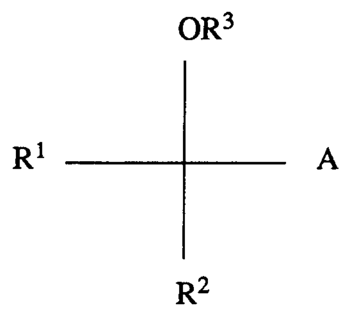

in welcher

$R^1$, $R^2$, $R^3$ und A die in der Beschreibung angegebenen Bedeutungen haben,

Verfahren zu ihrer Herstellung und ihre Verwendung als Zwischenprodukte bei der Synthese von Peptiden und Peptid-Mimetika.

EP 0 549 927 A2

Die Erfindung betrifft ein neues Verfahren zur Herstellung von neuen heterocyclisch substituierten Carbamaten, die wichtige Zwischenprodukte zur Synthese von pharmazeutischen Wirkstoffen, insbesondere zur Synthese von biologisch aktiven Peptiden und Peptid-Mimetika sind.

Gegenstand der Erfindung sind auch neue heterocyclisch substituierte Carbamate der allgemeinen Formel (I)

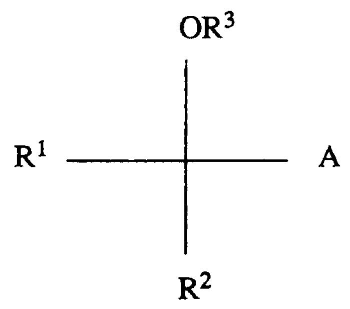

(I),

in welcher

$R^2$ für einen 3- bis 8-gliedrigen, gesättigten oder ungesättigten Heterocyclus mit bis zu 2 Heteroatomen aus der Reihe S oder O oder mit einer Gruppe der Formel-$NR^4$, -$SiR^5R^6R^7$ oder -$SnR^5R^6R^7$ steht,

worin

$R^4$ Wasserstoff, geradkettiges oder verzweigtes Alkyl mit bis zu 8 Kohlenstoffatomen, Phenyl oder Benzyl bedeutet,

$R^5$, $R^6$ und $R^7$ gleich oder verschieden sind und geradkettiges oder verzweigtes Alkyl mit bis zu 8 Kohlenstoffatomen oder Phenyl bedeuten,

wobei dieser gegebenenfalls bis zu 3-fach gleich oder verschieden durch geradkettiges oder verzweigtes Alkyl oder Alkoxy mit jeweils bis zu 8 Kohlenstoffatomen, Hydroxy, Phenoxy, Benzyl oder durch einen 5- bis 7-gliedrigen, gesättigten oder ungesättigten Heterocyclus mit bis zu 3 Heteroatomen aus der Reihe S, N oder O, durch Aryl mit 6 bis 10 Kohlenstoffatomen oder durch eine Gruppe der Formel -$NR^8R^9$ substituiert sein kann,

worin

$R^8$ und $R^9$ gleich oder verschieden sind und die oben angegebene Bedeutung von $R^4$ haben,

$R^1$ für Wasserstoff oder für die Gruppe -$SiR^5R^6R^7$ steht,

worin

$R^5$, $R^6$ und $R^7$ die oben angegebene Bedeutung haben,

$R^3$ für eine Gruppe der Formel -$CO-NR^{10}R^{11}$ steht,

worin

$R^{10}$ und $R^{11}$ gemeinsam mit dem Stickstoffatom einen heterocyclischen Rest der Formel

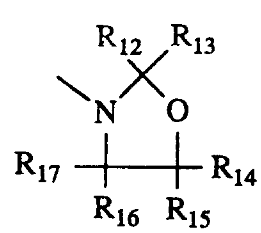

bilden,

worin

$R^{12}$, $R^{13}$, $R^{14}$, $R^{15}$, $R^{16}$ und $R^{17}$ gleich oder verschieden sind und Wasserstoff, geradkettiges oder verzweigtes Alkyl mit bis zu 8 Kohlenstoffatomen, Phenyl oder Cycloalkyl mit 3 bis 6 Kohlenstoffatomen bedeuten oder jeweils $R^{12}$ und $R^{13}$, $R^{14}$ und $R^{15}$ und/oder $R^{16}$ und $R^{17}$ gemeinsam einen 3- bis 6-gliedrigen, gesättigten Carbocyclus bilden,

A für geradkettiges oder verzweigtes Alkyl oder Alkenyl mit jeweils bis zu 8 Kohlenstoffatomen steht, die gegebenenfalls bis zu 3-fach gleich oder verschieden durch Hydroxy, Phenyl oder Cycloalkyl mit 3 bis 7 Kohlenstoffatomen substituiert sind oder durch eine Gruppe der Formel $-NR^{18}R^{19}$,$-HN-CO-OR^{20}$, $-SiR^{21}R^{22}R^{23}$ oder $-SnR^{21}R^{22}R^{23}$ substituiert sind,
worin
$R^{18}$ und $R^{19}$ die oben angegebene Bedeutung von $R^4$ haben und mit dieser gleich oder verschieden sind
und
$R^{20}$ geradkettiges oder verzweigtes Alkyl mit bis zu 6 Kohlenstoffatomen bedeutet, das gegebenenfalls durch Phenyl substituiert ist,
$R^{21}$, $R^{22}$ und $R^{23}$ die oben angegebene Bedeutung von $R^5$, $R^6$ und $R^7$ haben und mit dieser gleich oder verschieden sind,
oder
für geradkettiges oder verzweigtes Acyl mit bis zu 8 Kohlenstoffatomen steht,
für Cycloalkyl oder Cycloalkenyl mit 3 bis 7 Kohlenstoffatomen steht, das gegebenenfalls durch Hydroxy substituiert ist, oder
für Carboxy, Alkoxycarbonyl mit bis zu 4 Kohlenstoffatomen oder für eine Gruppe der Formel $-SiR^{21}R^{22}R^{23}$, $-SnR^{21}R^{22}R^{23}$ oder $R^{24}-CO-$ steht,
worin
$R^{21}$, $R^{22}$ und $R^{23}$ die oben angegebene Bedeutung haben
und
$R^{24}$ Wasserstoff oder geradkettiges oder verzweigtes Alkyl oder Alkenyl mit jeweils bis zu 8 Kohlenstoffatomen bedeutet, die gegebenenfalls bis zu 3-fach durch Phenyl oder durch die Gruppe der Formel $-NH-CO-OR^{20}$ substituiert sind,
worin
$R^{20}$ die oben angegebene Bedeutung hat.

Heterocyclus steht im allgemeinen in der Untersubstitution für einen 5- bis 7-gliedrigen, bevorzugt 5- bis 6-gliedrigen, gesättigten oder ungesättigten Ring, der als Heteroatome bis zu 2 Sauerstoff-, Schwefel- und/oder Stickstoffatome enthalten kann. Bevorzugt sind 5- und 6-gliedrige Ringe mit einem Sauerstoff-, Schwefel und/oder bis zu 2 Stickstoffatomen. Besonders bevorzugt werden genannt: Pyrrolyl, Pyrazolyl, Pyridyl, Pyrimidyl, Pyrazinyl, Pyridazinyl, Thiazolyl, Oxazolyl, Imidazolyl, Isoxazolyl, Pyrrolidinyl, Piperidinyl, Piperazinyl, Tetrazolyl oder Morpholinyl.

Für einen 3- bis 8-gliedrigen, gesättigten Heterocyclus stehen im allgemeinen Pyrrolidin, Piperidin, Pyrazolidin, Imidazolidin oder Oxazolan. Bevorzugt sind Pyrrolidin und Oxazolan.

Bevorzugt sind Verbindungen der allgemeinen Formel (I),
in welcher
$R^2$ für einen heterocyclischen Rest der Formel

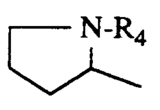

oder

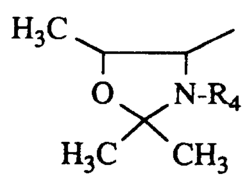

steht,
worin
$R^4$ Wasserstoff, geradkettiges oder verzweigtes Alkyl mit bis zu 6 Kohlenstoffatomen, Phenyl oder Benzyl bedeutet,
$R^1$ für Wasserstoff steht,
$R^3$ für eine Gruppe der Formel $-CO-NR^{10}R^{11}$ steht,
worin
$R^{10}$ und $R^{11}$ gemeinsam mit dem Stickstoffatom einen heterocyclischen Rest der

Formel

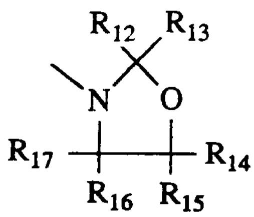

bilden,

worin

$R^{12}$, $R^{13}$, $R^{14}$, $R^{15}$, $R^{16}$ und $R^{17}$ gleich oder verschieden sind und Wasserstoff, geradkettiges oder verzweigtes Alkyl mit bis zu 6 Kohienstoffatomen, Cyclopropyl, Cyclopentyl oder Cyclohexyl bedeuten, oder jeweils $R^{12}$ und $R^{13}$, $R^{14}$ und $R^{15}$ oder $R^{16}$ und $R^{17}$ gemeinsam einen Cyclopropyl-, Cyclopentyl- oder Cyclohexylring bilden,

A für geradkettiges oder verzweigtes Alkyl oder Alkenyl mit jeweils bis zu 6 Kohlenstoffatomen steht, die gegebenenfalls bis zu 2-fach gleich oder verschieden durch Hydroxy, Phenyl, Cyclobutyl, Cyclopentyl, Cyclohexyl oder durch eine Gruppe der Formel $-NR^{18}R^{19}$, -NH-CO-$OR^{20}$, $-SiR^{21}R^{22}R^{23}$ oder $-SnR^{21}R^{22}R^{23}$ substituiert sind,

worin

$R^{18}$ und $R^{19}$ die oben angegebene Bedeutung von $R^4$ haben und mit dieser gleich oder verschieden sind,

$R^{20}$ geradkettiges oder verzweigtes Alkyl mit bis zu 4 Kohlenstoffatomen bedeutet, das gegebenenfalls durch Phenyl substituiert ist,

$R^{21}$, $R^{22}$ und $R^{23}$ gleich oder verschieden sind und geradkettiges oder verzweigtes Alkyl mit bis zu 4 Kohlenstoffatomen oder Phenyl bedeuten,

oder

für geradkettiges oder verzweigtes Acyl mit bis zu 6 Kohlenstoffatomen steht,

für Cyclobutyl oder Cyclohexyl steht, die gegebenenfalls durch Hydroxy substituiert sind, oder

für Carboxy, Alkoxycarbonyl mit bis zu 3 Kohlenstoffatomen oder für eine Gruppe der Formel $-SiR^{21}R^{22}R^{23}$, $-SnR^{21}R^{22}R^{23}$ oder $R^{24}$-CO-steht,

worin

$R^{21}$, $R^{22}$ und $R^{23}$ die oben angegebene Bedeutung haben

und

$R^{24}$ Wasserstoff oder geradkettiges oder verzweigtes Alkyl oder Alkenyl mit jeweils bis zu 6 Kohlenstoffatomen bedeutet, die gegebenenfalls durch Phenyl oder durch die Gruppe der Formel -NH-CO-$OR^{20}$ substituiert sind,

worin

$R^{20}$ die oben angegebene Bedeutung hat.

Besonders bevorzugt sind Verbindungen der allgemeinen Formel (I),

in welcher

$R^2$ für einen heterocyclischen Rest der Formel

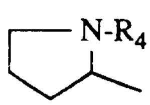

oder

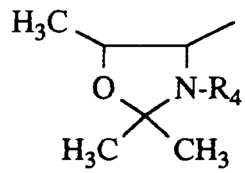

steht,
worin

$R^4$ Wasserstoff, geradkettiges oder verzweigtes Alkyl mit bis zu 4 Kohlenstoffatomen, Phenyl oder Benzyl bedeutet,

$R^1$ für Wasserstoff steht,

$R^3$ für eine Gruppe der Formel $-CO-NR^{10}R^{11}$ steht,
worin

$R^{10}$ und $R^{11}$ gemeinsam mit dem Stickstoffatom einen heterocyclischen Ring der Formel

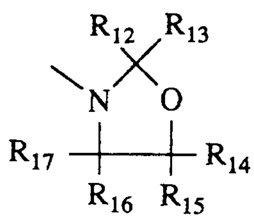

bilden,
worin

$R^{12}$, $R^{13}$, $R^{14}$, $R^{15}$, $R^{16}$ und $R^{17}$ gleich oder verschieden sind und Wasserstoff, geradkettiges oder verzweigtes Alkyl mit bis zu 4 Kohlenstoffatomen, Cyclopropyl, Cyclopentyl oder Cyclohexyl bedeuten, oder jeweils $R^{12}$ und $R^{13}$, $R^{14}$ und $R^{15}$ oder $R^{16}$ und $R^{17}$ gemeinsam einen Cyclopropyl-, Cyclopentyl- oder Cyclohexylring bilden,

A für geradkettiges oder verzweigtes Alkyl oder Alkenyl mit jeweils bis zu 4 Kohlenstoffatomen steht, die gegebenenfalls durch Hydroxy, Phenyl, Cyclobutyl, Cyclohexyl oder durch eine Gruppe der Formel $-NR^{18}R^{19}$, $-NHCO-OR^{20}$, $-SiR^{21}R^{22}R^{23}$ oder $-SnR^{21}R^{22}R^{23}$ substituiert sind,
worin

$R^{18}$ und $R^{19}$ gleich oder verschieden sind und die oben angegebene Bedeutung von $R^4$ haben und mit dieser gleich oder verschieden sind,

$R^{20}$ Methyl oder Ethyl bedeutet, die gegebenenfalls durch Phenyl substituiert sind,

$R^{21}$, $R^{22}$ und $R^{23}$ geradkettiges oder verzweigtes Alkyl mit bis zu 4 Kohlenstoffatomen bedeuten,

oder

für geradkettiges oder verzweigtes Acyl mit bis zu 4 Kohienstoffatomen steht,

für Cyclobutyl oder Cyclohexyl steht, die gegebenenfalls durch Hydroxy substituiert sind,

für Carboxy, Alkoxycarbonyl mit bis zu 3 Kohienstoffatomen oder für eine Gruppe der Formel $-SiR^{21}R^{22}R^{23}$, $-SnR^{21}R^{22}R^{23}$ oder $R^{24}-CO-$ steht,
worin

$R^{21}$, $R^{22}$ und $R^{23}$ die oben angegebene Bedeutung haben
und

$R^{24}$ Wasserstoff oder geradkettiges oder verzweigtes Alkyl oder Alkenyl

mit jeweils bis zu 4 Kohlenstoffatomen bedeutet, die gegebenenfalls durch Phenyl oder durch die Gruppe der Formel -NH-CO-$OR^{20}$ substituiert sind,
worin

$R^{20}$ die oben angegebene Bedeutung hat.

Außerdem wurde ein neues Verfahren zur Herstellung der erfindungsgemäßen Verbindungen der allgemeinen Formel (I) gefunden, dadurch gekennzeichnet, daß man
Carbamate der allgemeinen Formel (II)

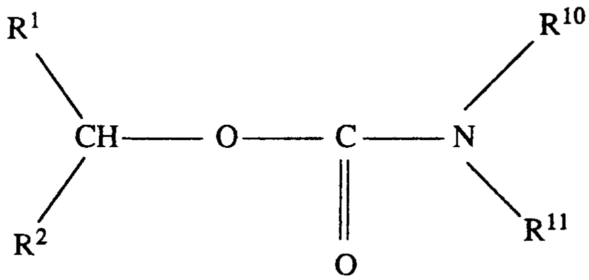 (II),

in welcher
$R^1$, $R^2$, $R^{10}$ und $R^{11}$ die oben angegebene Bedeutung haben,
zunächst in inerten Lösemitteln, in Anwesenheit einer selektiven Base, vorzugsweise sec.-Butyllithium, und eines chelatbildenden Diamins (im folgenden mit D bezeichnet) zu den Carbanion-Komplexverbindungen der allgemeinen Formel (III)

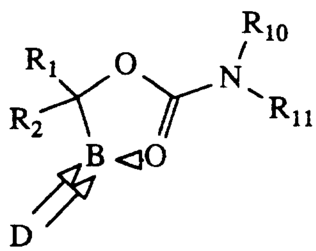 (III),

in welcher
$R^1$, $R^2$, $R^{10}$ und $R^{11}$ die oben angegebene Bedeutung haben,
B für ein Lithium-, Magnesium-, Titan-, Zirkonium- oder Kaliumatom, vorzugsweise für Lithium, steht,
und
D für ein chelatbildendes Diamin wie beispielsweise Tetramethylethylendiamin (TMEDA) oder (-)-Spartein, vorzugsweise TMEDA, steht,
enantioselektiv deprotoniert und anschließend mit Elektrophilen der allgemeinen Formeln (IV) und (V)

A-M (IV)

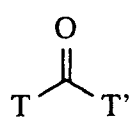 (V),

in welcher
A die oben angegebene Bedeutung hat,
M für Halogen, für $C_1$-$C_4$-Alkoxy oder für eine andere typische Abgangsgruppe, vorzugsweise für Chlor, steht,
und

T und T' gleich oder verschieden sind und für Wasserstoff oder für einen unter dem Substituenten A aufgeführten chemisch sinnvollen Rest stehen,

oder mit $CO_2$ umsetzt.

Das erfindungsgemäße Verfahren kann beispielhaft durch folgendes Formelschema erläutert werden:

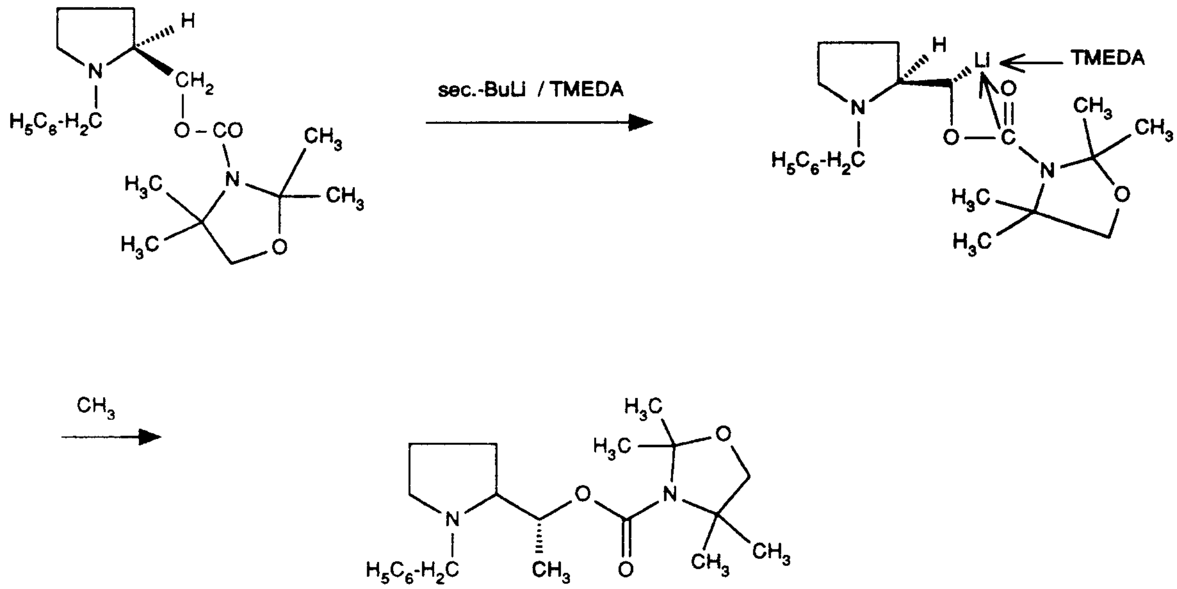


Überraschenderweise liefert das erfindungsgemäße Verfahren die gewünschten Verbindungen der allgemeinen Formel (I) in guten Ausbeuten.

Das Verfahren zeichnet sich durch mehrere Vorteile aus: im Gegensatz zum Stand der Technik sind stöchiometrische Mengen an Basen, vorzugsweise sec.-Butyllithium für die Deprotonierung ausreichend. Außerdem ermöglicht das erfindungsgemäße Verfahren sowohl eine Steuerung der Diastereoselektivität durch die Wahl der elektrophilen Substituenten als auch die Beeinflussung der asymmetrischen Induktion bei prochiralen Carbamaten in Gegenwart von chiralen komplexbildenden Diaminen.

Durch Einsatz von enantiomerenreinen Diaminverbindungen wie beispielsweise TMEDA, erfolgt die Deprotonierung der Verbindungen der Formel (II) enantioselektiv und in sehr guten Ausbeuten zu den entsprechenden chiralen, bevorzugt zu den (S)-Lithiumverbindungen der Formel (III), die durch weitere Umsetzung mit Elektrophilen, bevorzugt in der R-Konfiguration überführt werden können.

Es ist außerdem bei Kenntnis des Standes der Technik überraschend, daß die metallierten Verbindungen der allgemeinen Formel (III) keiner Zersetzung und einer Deprotonierung im Benzylrest unterliegen. Insbesondere ist eine hohe Diastereoselektivität und die stereochemische Lenkung bei dem Angriff des Elektrophils durch die Induktion des bereits vorhandenen Stereozentrums von großem Vorteil.

Neben Chlor, Brom und Jod werden unter der Definition Abgangsgruppe auch die Reste Tosylat, Mesylat oder $-OSO_2CF_3$ erfaßt. Bevorzugt ist Chlor.

Als Lösemittel für die Deprotonierung eignen sich bevorzugt inerte organische Lösemittel wie Kohlenwasserstoffe wie Hexan, Pentan, Ligroin oder Toluol, und Ether, beispielsweise Tetrahydrofuran, Diethylether, Dioxan, Dimethoxyethan, Diglyme, Triglyme oder tert.-Butylmethylether. Besonders bevorzugt sind Tetrahydrofuran und Diethylether.

Die Deprotonierung erfolgt in einem Temperaturbereich von -100 °C bis Raumtemperatur, vorzugsweise bei ca. -78 °C bis 0 °C.

Die Deprotonierung kann sowohl bei Normaldruck als auch bei erhöhtem oder erniedrigtem Druck (z.b. 0,5 bis 2 bar), vorzugsweise bei Normaldruck, durchgeführt werden.

Als selektive Basen eignen sich Alkyllithiumverbindungen mit bis zu 6 C-Atomen in der Alkylgruppe, vorzugsweise n-Butyllithium oder sec.-Butyllithium.

Die Base wird in einer Menge von 0,5 bis 5 mol, vorzugsweise in stöchiometrischen Mengen, bezogen auf 1 Mol der Verbindungen der allgemeinen Formel (II), eingesetzt.

Die elektrophile Substitution erfolgt ebenfalls in den oben aufgeführten Losemitteln, bevorzugt in Tetrahydrofuran bei Normaldruck.

Die elektrophile Substitution wird in einem Temperaturbereich von ca. -100 °C bis +40 °C, vorzugsweise im Bereich von -78 °C bis Raumtemperatur durchgeführt.

Die Verbindungen der allgemeinen Formel (II) sind ebenfalls neu und können hergestellt werden, indem man

Verbindungen der allgemeinen Formel (VI)

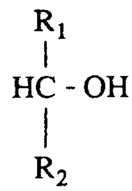 (VI),

in welcher

$R^1$ und $R^2$ die oben angegebene Bedeutung haben

mit Verbindungen der allgemeinen Formel (VII)

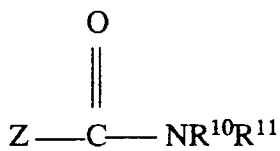 (VII),

in welcher

$R^{10}$ und $R^{11}$ die oben angegebene Bedeutung haben

und

Z für Halogen, vorzugsweise für Chlor, steht,

in einem der oben aufgeführten Losemittel, vorzgusweise Ether, in Anwesenheit einer Base, vorzugsweise Natriumhydrid, umsetzt.

Die Verbindungen der allgemeinen Formeln (VI) und (VII) sind bekannt oder können nach üblicher Methode hergestellt werden [vgl. z.B. J. Chem. Soc., Perkin Trans 1, 1074, 1101].

Die Verbindungen der allgemeinen Formel (III) sind neu und können nach dem oben angegebenen Verfahren hergestellt werden.

Die Verbindungen der allgemeinen Formeln (IV) und (V) sind bekannt oder nach üblichen Methoden herstellbar.

Die erfindungsgemäßen Verbindungen zeichnen sich auch dadurch aus, daß nach der enantioselektiven Einführung des Elektrophils, die Schutzgruppe $-CO-NR^{10}R^{11}$ unter Ausbildung der freien Hydroxygruppe sehr leicht abgespalten werden kann.

Die Abspaltung der Schutzgruppen (Carbamidsäureester) erfolgt nach üblicher Methode z.B. durch sequentielle Behandlung mit Säuren und Basen bevorzugt in Methanol.

Als Säuren eignen sich starke anorganische Säuren und organische Sulfon- oder Carbonsäuren wie beispielsweise Methansulfonsäure, Ethansulfonsäure, Benzolsulfonsäure, Toluolsulfonsäure, Essigsäure oder Propionsäure.

Als Basen eignen sich Alkali- und Erdalkalihydroxide wie beispielsweise Natrium-, Kalium- oder Bariumhydroxid. Bevorzugt ist Bariumhydroxid.

Die Säuren und Basen werden in einer Menge von 0,01 bis 10 mol, vorzugsweise 1 mol, bezogen auf 1 mol der Verbindungen der allgemeinen Formel (I), eingesetzt.

Die Abspaltung der Schutzgruppen erfolgt bei Normaldruck in einem Temperaturbereich von 0 °C bis +130 °C, vorzugsweise von +20 °C bis +100 °C.

Die Abspaltung kann auch mit Lithiumaluminiumhydrid in einem der oben aufgeführten Lösemittel, vorzugsweise Tetrahydrofuran, erfolgen.

Die erfindungsgemäßen Verbindungen sind somit wertvolle Zwischenprodukte zur Herstellung von teilweise bekannten oder neuen Hydroxy-substituierten Heterocyclen, insbesondere Prolinderivaten, die zur Synthese von biologisch aktiven Peptiden oder Peptid-Mimetika von großer Bedeutung sind.

Die im folgenden benutzten Abkürzungen a und b haben die folgende Bedeutung:

a = 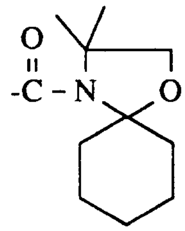

b = 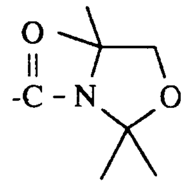

Ausgangsverbindungen

Beispiel I

(S)-2-(Hydroxymethyl)-1-benzyl-pyrrolidin

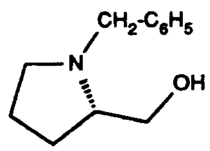

3,79 g (37,5 mmol) (S)-Prolinol und 5,5 ml (47,4 mmol) Benzylchlorid werden mit 3,7 g Kaliumcarbonat in 100 ml Toluol 48 h unter Rückfluß erhitzt. Entgegen der Literaturvorschrift wird im Vakuum fraktioniert. Man erhält 5,52 g (77%, Lit.: 66%) der Titelverbindung als farbloses Öl. Die spektroskopischen Daten entsprechen denen in der Literatur angegebenen.

Beispiel II

(S)-(-)-[(1-Benzylpyrrolidinyl)-methyl]-2,2,4,4-tetramethyl-1,3-oxazolidin-3-carboxylat

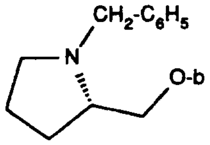

Bei Raumtemperatur werden in 10 ml Ether 402 mg (14 mmol) Natriumhydrid (80%ig in Mineralöl) vorgelegt. Hierzu tropft man 1,91 g (10 mmol) der Verbindung aus Beispiel I. Es wird bis zur vollständigen Deprotonierung 1 h gerührt und dann mit 2,30 g (12 mmol) des Carbamoylchlorides, gelöst in 5 ml Ether, versetzt. Die Reaktionsmischung rührt man 5 Tage bei Raumtemperatur. Die Hydrolyse erfolgt anschließend mit 5 ml Wasser. Die wäßrige Phase wird dreimal mit je 5 ml Ether extrahiert, die vereinigten organischen Phasen werden über Magnesiumsulfat getrocknet und im Vakuum eingeengt. Nach säulenchromatographischer Trennung an Kieselgel (Ether / Pentan = 1:2, Kieselgel 0,2 - 0,06) erhält man 2,84 g (82%) der Titelverbindung als farbloses Öl.
$R_f$ = 0,37 (Kieselgel, Ether / Pentan = 1:1)
$[\alpha]_D^{20}$ = -60,2 (c = 2.1, $CHCl_3$).

Herstellungsbeispiele

Beispiel 1

(1R,2'S)-(-)-[(1-Benzylpyrrolidinyl)ethyl]2,2,4,4-tetramethyl-1,3-oxazolidin-3-carboxylat

a) Vorstufe

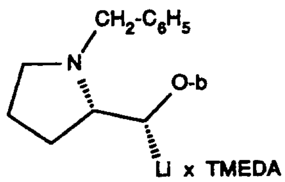

b)

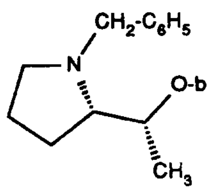

In 8 ml Ether werden 346 mg (1,0 mmol) der Verbindung aus Beispiel II und 446 µl Tetramethylethylendiamin (TMEDA, 3,0 mmol) gelöst. Diese Lösung wird bei -78 °C mit 2,17 ml (3,0 mmol) sec.-Butyllithium (1,4M in Cyclohexan / Isopentan) versetzt; dabei verfärbt sich die Lösung orangegelb (a). Bei dieser Temperatur wird 3 h gerührt und dann 187 µl (3,0 mmol) Iodmethan injiziert. Es wird noch 1 h bei dieser Temperatur nachgerührt und dann das Kältebad entfernt. Nach Auftauen auf Raumtemperatur wird die Lösung mit 2 ml Wasser hydrolysiert. Nach Trennung der Phasen wird die wäßrige noch dreimal mit je 5 ml Ether extrahiert. Die vereinigten organischen Phasen werden über Magnesiumsulfat getrocknet und im Vakuum eingeengt. Nach säulenchromatographischer Trennung an Aluminiumhydroxid (Aktivitätsstufe II) können 259 mg (72%) der Titelverbindung als farblose Kristalle isoliert werden.
Schmp.: 52 °C (aus der Schmelze)
$R_f$ = 0,67 (Aluminiumoxid, Ether / Pentan = 1:1)
$[\alpha]_D^{20}$ = -51,0 (c = 0,5, $CHCl_3$).

Beispiel 2

(1R,2'S)-(-)-[(1-Benzylpyrrolidinyl)-trimethylsilyl-methyl]-2,2,4,4,-tetramethyl-1,3-oxazolidin-3-carboxylat

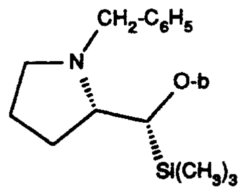

Nach der oben beschriebenen Methode erhält man bei der Umsetzung von 346 mg (1,0 mmol) der Verbindung aus Beispiel II mit 2,17 ml (3,0 mmol) sec.-Butyllithium, 446 µl (3,0 mmol) TMEDA und 378 µl Trimethylsilylchlorid 140 mg (33%) der Titelverbindung als wachsartigen Feststoff. Schmp.: 45 °C (aus der Schmelze)
$R_f$ = 0,55 (Aluminiumoxid, Ether / Pentan = 1:1)
$[\alpha]_D^{20}$ = -54,8 (c = 1,5, $CHCl_3$).

Beispiel 3

(1R,2'S)-(-)-[(1-Benzylpyrrolidinyl)tributylstannyl-methyl]-2,2,4,4-tetramethyl-1,3-oxazolidin-3-carboxylat

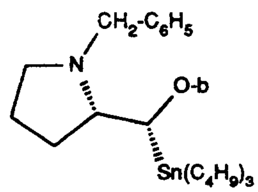

Analog erhält man aus 346 mg (1,0 mmol) der Verbindung aus Beispiel II und 865 µl (3,0 mmol) Tributylstannylchlorid 451 mg (71%) der Titelverbindung als farbloses Öl.
$R_f$ = 0,66 (Aluminiumoxid, Ether / Pentan = 1:1)
$[\alpha]_D^{20}$ = -69,6 (c = 1,0, $CHCl_3$).

Beispiel 4

(2S,2'S)-(-)-2-[1-Benzylpyrrolidinyl-(2,2,4,4-tetramethyl-1,3-oxazolidin-3-yl-carbonyl)oxy]-ethansäuremethylester

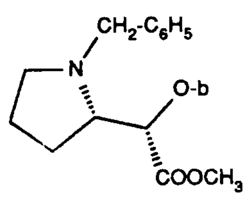

346 mg (1,0 mmol) der Verbindung aus Beispiel II werden nach der oben beschriebenen Methode 3 h metalliert. Anschließend wird bei -78 °C 1 h Kohlendioxid eingeleitet und unter Kohlendioxidatmosphäre auf Raumtempertur erwärmt. Die Reaktionslösung wird mit 10 ml 2 N Salzsäure hydrolysiert. Die wäßrige Phase wird noch dreimal mit je 10 ml Ether extrahiert. Die vereinigten etherischen Phasen werden über Magnesiumsulfat getrocknet und im Vakuum eingeengt. Der Rückstand wird in 10 ml Ether gelöst und mit

Diazomethan (0,6M in Ether) bis zur bleibenden Gelbfärbung versetzt; überschüssiges Diazomethan wird mit Kieselgel vernichtet. Die Lösung wird im Vakuum vom Lösemittel befreit und säulenchromatographisch über Kieselgel (Ether / Pentan = 1:4) gereinigt. Man erhält 162 mg (40%) der Titelverbindung als farbloses Öl.
$R_f$ = 0,46 (Kieselgel, Ether / Pentan = 1:1)
$[\alpha]_D^{20}$ = -19,2 (c = 1,0, $CHCl_3$)

Beispiel 5

(1S;2'S)-(-)-[1-(1-Benzylpyrrolidinyl)-2-hydroxymethylpropyl]-2,2,4,4-tetramethyl-1,3-oxazolidin-3-carboxylat

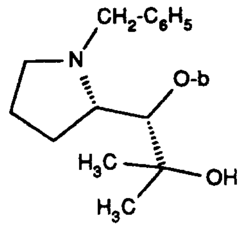

Bei der Metallierung von 346 mg (1,0 mmol) der Verbindung aus Beispiel II mit 446 µl (3,0 mmol) TMEDA und 2,17 ml (3,0 mmol) sec.-Butyllithium und anschließender Umsetzung mit 220 µl (3,0 mmol) Aceton ergeben sich 135 mg (35%) der Titelverbindung als farblose Kristalle. Schmp.: 102 °C (Ether / Pentan)
$R_f$ = 0,20 (Kieselgel, Ether / Pentan = 1:1)

$$[\alpha]_{365}^{20} = -17{,}4 \ (c = 1{,}0, CHCl_3).$$

Beispiel 6

(1S,2RS, 2'S)-(-)-[1-(1-Benzylpyrrolidinyl)-2-hydroxy-3-methylbutyl]-2,2,4,4-tetramethyl-1,3-oxazolidin-3-carboxylat

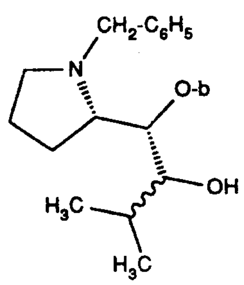

Aus der Umsetzung von 346 mg (1,0 mmol) der Verbindung aus Beipiel II mit 273 µl (3,0 mmol) Isobutyraldehyd erhält man zwei Diastereomere. Das eine Diastereomer isoliert man als farblose Kristalle in 23% (95 mg) Ausbeute.
Schmp.: 64 °C
$R_f$ = 0,52 (Aluminiumhydroxid, Ether / Pentan = 1:1)
$[\alpha]_D^{20}$ = -8,1;

$$[\alpha]^{20}_{365} = -13,7 \ (c = 1,2; CHCl_3)$$

Das zweite Diastereomer erhält man als farbloses Öl in 62% (260 mg) Ausbeute.
$R_f$ = 0,43 (Aluminiumhydroxid, Ether / Pentan = 1:1)
$[\alpha]^{20}_{D}$ = -1,3;

$$[\alpha]^{20}_{365} = -15,8 \ (c = 1,2; CHCl_3).$$

Mit den Beispielen A und B soll beispielhaft erläutert werden wie die erfindungsgemäßen Verbindungen der allgemeinen Formel (I) in die entsprechenden Hydroxyverbindungen überführt werden können.

Beispiel A

(1R, 2'S)-(-)-(1-Benzylpyrrolidinyl)-ethanol

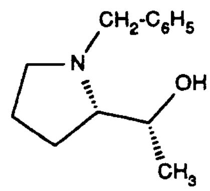

1,41 g (3,9 mmol) der Verbindung aus Beispiel 1 werden mit 740 µl (11,4 mmol) Methansulfonsäure in 15 ml Methanol 16 h unter Rückfluß erhitzt. Anschließend wird nach Zugabe von 5 g Bariumhydroxid weitere 4 h unter Rückfluß erhitzt. Nach Abkühlen auf Raumtemperatur wird der anorganische Rückstand über eine kurze Kieselgelsäule abfiltriert. Das Filtrat wird im Vakuum eingeengt, mit Ether aufgenommen und über Magnesiumsulfat getrocknet. Nach Entfernen des Lösemittels im Vakuum wird das zurückbleibende Öl über Aluminiumoxid (Aktivitätsstufe III, Ether / Pentan = 1:2) gereinigt. Man erhält 532 mg (66%) der Titelverbindung als farbloses, leicht zersetzliches Öl.
$R_f$ = 0,56 (Aluminiumoxid, Ether / Pentan = 1:1)
$[\alpha]^{20}_{D}$ = -85,0 (c = 0,7, $CHCl_3$).

Beispiel B

(2S, 3S)-(-)-2-(Hydroxyethyl)-pyrrolidin

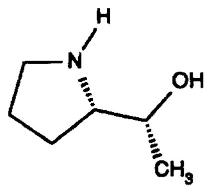

Zum Entfernen der Benzylgruppe werden 481 mg (2,3 mmol) der Verbindung aus Beispiel A mit 488 mg (0,46 mmol) Palladium (10%ig auf Kohle) in 15 ml Methanol und 0,5 ml Ameisensäure 8 h unter leichtem Wasserstoffüberdruck hydriert. Nach Ende der Reaktion wird das Palladium über eine dünne Kieselgelschicht abfiltriert. Das Filtrat wird im Vakuum eingeengt und mit 5 ml 5 N Salzsäure aufgenommen. Die salzsaure Lösung wird dreimal mit je 5 ml Ether extrahiert und die Extrakte verworfen. Nach vorsichtiger Neutralisation mit 30%iger Natronlauge wird die wäßrige Phase dreimal mit je 10 ml Essigester extrahiert. Die vereinigten organischen Essigesterphasen werden über Magnesiumsulfat getrocknet und im Vakuum vom Lösemittel befreit. Der Rückstand wird aus Essigester / Pentan umkristallisiert. Man erhält so 112 mg

(42%) der Titelverbindung als leicht zersetzliche, farblose Kristalle.
Schmp.: 86 ° C
$[\alpha]_D^{20}$ = -36,4 (c = 1,0, $CH_3OH$).

## Patentansprüche

**1.** Heterocyclisch substituierte Carbamate der allgemeinen Formel (I)

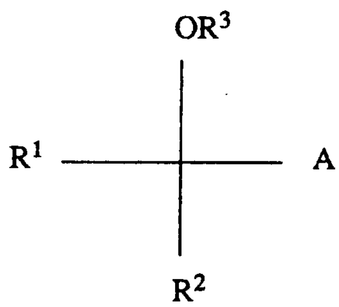 (I),

in welcher

| | |
|---|---|
| $R^2$ | für einen 3- bis 8-gliedrigen, gesättigten oder ungesättigten Heterocyclus mit bis zu 2 Heteroatomen aus der Reihe S oder O oder mit einer Gruppe der Formel-$NR^4$, -$SiR^5R^6R^7$ oder -$SnR^5R^6R^7$ steht, |
| | worin |
| $R^4$ | Wasserstoff, geradkettiges oder verzweigtes Alkyl mit bis zu 8 Kohlenstoffatomen, Phenyl oder Benzyl bedeutet, |
| $R^5$, $R^6$ und $R^7$ | gleich oder verschieden sind und geradkettiges oder verzweigtes Alkyl mit bis zu 8 Kohlenstoffatomen oder Phenyl bedeuten, |
| | wobei dieser gegebenenfalls bis zu 3-fach gleich oder verschieden durch geradkettiges oder verzweigtes Alkyl oder Alkoxy mit jeweils bis zu 8 Kohlenstoffatomen, Hydroxy, Phenoxy, Benzyl oder durch einen 5- bis 7-gliedrigen, gesättigten oder ungesättigten Heterocyclus mit bis zu 3 Heteroatomen aus der Reihe S, N oder O, durch Aryl mit 6 bis 10 Kohlenstoffatomen oder durch eine Gruppe der Formel -$NR^8R^9$ substituiert sein kann, |
| | worin |
| $R^8$ und $R^9$ | gleich oder verschieden sind und die oben angegebene Bedeutung von $R^4$ haben, |
| $R^1$ | für Wasserstoff oder für die Gruppe -$SiR^5R^6R^7$ steht, |
| | worin |
| $R^5$, $R^6$ und $R^7$ | die oben angegebene Bedeutung haben, |
| $R^3$ | für eine Gruppe der Formel -$CO$-$NR^{10}R^{11}$ steht, |
| | worin |
| $R^{10}$ und $R^{11}$ | gemeinsam mit dem Stickstoffatom einen heterocyclischen Rest der Formel |

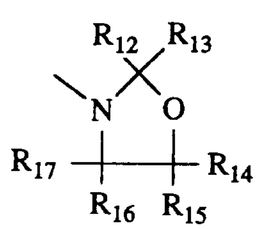

bilden,
worin

$R^{12}$, $R^{13}$, $R^{14}$, $R^{15}$, $R^{16}$ und $R^{17}$ gleich oder verschieden sind und Wasserstoff, geradkettiges oder verzweigtes Alkyl mit bis zu 8 Kohlenstoffatomen, Phenyl oder Cycloalkyl mit 3 bis 6 Kohlenstoffatomen bedeuten oder jeweils $R^{12}$ und $R^{13}$, $R^{14}$ und $R^{15}$ und/oder $R^{16}$ und $R^{17}$ gemeinsam einen 3- bis 6-gliedrigen, gesättigten Carbocyclus bilden,

A für geradkettiges oder verzweigtes Alkyl oder Alkenyl mit jeweils bis zu 8 Kohlenstoffatomen steht, die gegebenenfalls bis zu 3-fach gleich oder verschieden durch Hydroxy, Phenyl oder Cycloalkyl mit 3 bis 7 Kohlenstoffatomen substituiert sind oder durch eine Gruppe der Formel $-NR^{18}R^{19}$, $-HN-CO-OR^{20}$, $-SiR^{21}R^{22}R^{23}$ oder $-SnR^{21}R^{22}R^{23}$ substituiert sind,
worin

$R^{18}$ und $R^{19}$ die oben angegebene Bedeutung von $R^4$ haben und mit dieser gleich oder verschieden sind
und

$R^{20}$ geradkettiges oder verzweigtes Alkyl mit bis zu 6 Kohlenstoffatomen bedeutet, das gegebenenfalls durch Phenyl substituiert ist,

$R^{21}$, $R^{22}$ und $R^{23}$ die oben angegebene Bedeutung von $R^5$, $R^6$ und $R^7$ haben und mit dieser gleich oder verschieden sind,
oder
für geradkettiges oder verzweigtes Acyl mit bis zu 8 Kohlenstoffatomen steht,
für Cycloalkyl oder Cycloalkenyl mit 3 bis 7 Kohlenstoffatomen steht, das gegebenenfalls durch Hydroxy substituiert ist, oder
für Carboxy, Alkoxycarbonyl mit bis zu 4 Kohlenstoffatomen oder
für eine Gruppe der Formel $-SiR^{21}R^{22}R^{23}$, $-SnR^{21}R^{22}R^{23}$ oder $R^{24}$-CO- steht,
worin

$R^{21}$, $R^{22}$ und $R^{23}$ die oben angegebene Bedeutung haben
und

$R^{24}$ Wasserstoff oder geradkettiges oder verzweigtes Alkyl oder Alkenyl mit jeweils bis zu 8 Kohlenstoffatomen bedeutet, die gegebenenfalls bis zu 3-fach durch Phenyl oder durch die Gruppe der Formel $-NH-CO-OR^{20}$ substituiert sind,
worin

$R^{20}$ die oben angegebene Bedeutung hat.

**2.** Verbindungen der allgemeinen Formel (I) gemäß Anspruch 1, in welcher

$R^2$ für einen heterocyclischen Rest der Formel

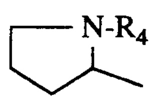

oder

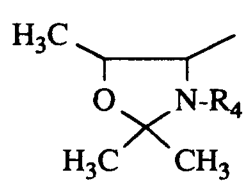

steht,
worin

$R^4$ Wasserstoff, geradkettiges oder verzweigtes Alkyl mit bis zu 6 Kohlenstoffatomen, Phenyl oder Benzyl bedeutet,

$R^1$ für Wasserstoff steht,

$R^3$ für eine Gruppe der Formel $-CO-NR^{10}R^{11}$ steht,
worin

$R^{10}$ und $R^{11}$ gemeinsam mit dem Stickstoffatom einen heterocyclischen Rest der Formel

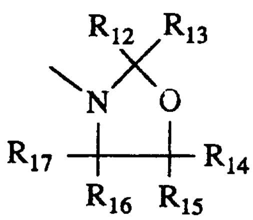

bilden,
worin

$R^{12}$, $R^{13}$, $R^{14}$, $R^{15}$, $R^{16}$ und $R^{17}$ gleich oder verschieden sind und Wasserstoff, geradkettiges oder verzweigtes Alkyl mit bis zu 6 Kohlenstoffatomen, Cyclopropyl, Cyclopentyl oder Cyclohexyl bedeuten, oder jeweils $R^{12}$ und $R^{13}$, $R^{14}$ und $R^{15}$ oder $R^{16}$ und $R^{17}$ gemeinsam einen Cyclopropyl-, Cyclopentyl- oder Cyclohexylring bilden,

A für geradkettiges oder verzweigtes Alkyl oder Alkenyl mit jeweils bis zu 6 Kohlenstoffatomen steht, die gegebenenfalls bis zu 2-fach gleich oder verschieden durch Hydroxy, Phenyl, Cyclobutyl, Cyclopentyl, Cyclohexyl oder durch eine Gruppe der Formel $-NR^{18}R^{19}$, $-NH-CO-OR^{20}$, $-SiR^{21}R^{22}R^{23}$ oder $-SnR^{21}R^{22}R^{23}$ substituiert sind,
worin

$R^{18}$ und $R^{19}$ die oben angegebene Bedeutung von $R^4$ haben und mit dieser gleich oder verschieden sind,

$R^{20}$ geradkettiges oder verzweigtes Alkyl mit bis zu 4 Kohlenstoffatomen bedeutet, das gegebenenfalls durch Phenyl substituiert ist,

$R^{21}$, $R^{22}$ und $R^{23}$ gleich oder verschieden sind und geradkettiges oder verzweigtes Alkyl mit bis zu 4 Kohlenstoffatomen oder Phenyl bedeuten,
oder
für geradkettiges oder verzweigtes Acyl mit bis zu 6 Kohlenstoffatomen steht,
für Cyclobutyl oder Cyclohexyl steht, die gegebenenfalls durch Hydroxy substituiert sind, oder
für Carboxy, für Alkoxycarbonyl mit bis zu 3 Kohlenstoffatomen oder für eine Gruppe der Formel $-SiR^{21}R^{22}R^{23}$, $-SnR^{21}R^{22}R^{23}$ oder $R^{24}-CO-$ steht,
worin

$R^{21}$, $R^{22}$ und $R^{23}$ die oben angegebene Bedeutung haben
und

$R^{24}$ Wasserstoff oder geradkettiges oder verzweigtes Alkyl oder Alkenyl mit jeweils bis zu 6 Kohlenstoffatomen bedeutet, die gegebenenfalls durch Phenyl oder durch die Gruppe der Formel $-NH-CO-OR^{20}$ substituiert sind,
worin

$R^{20}$ die oben angegebene Bedeutung hat.

3. Verbindungen der allgemeinen Formel (I) gemaß Anspruch 1, in welcher

$R^2$ für einen heterocyclischen Rest der Formel

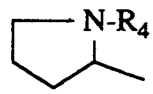

oder

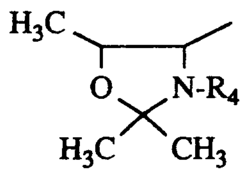

steht,

worin

$R^4$ Wasserstoff, geradkettiges oder verzweigtes Alkyl mit bis zu 4 Kohlenstoffatomen, Phenyl oder Benzyl bedeutet,

$R^1$ für Wasserstoff steht,

$R^3$ für eine Gruppe der Formel $-CO-NR^{10}R^{11}$ steht,

worin

$R^{10}$ und $R^{11}$ gemeinsam mit dem Stickstoffatom einen heterocyclischen Ring der Formel

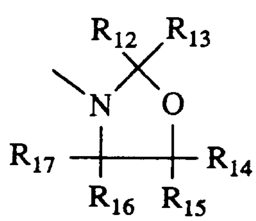

bilden,

worin

$R^{12}$, $R^{13}$, $R^{14}$, $R^{15}$, $R^{16}$ und $R^{17}$ gleich oder verschieden sind und Wasserstoff, geradkettiges oder verzweigtes Alkyl mit bis zu 4 Kohlenstoffatomen, Cyclopropyl, Cyclopentyl oder Cyclohexyl bedeuten, oder jeweils $R^{12}$ und $R^{13}$, $R^{14}$ und $R^{15}$ oder $R^{16}$ und $R^{17}$ gemeinsam einen Cycloproyl-, Cyclpentyl- oder Cyclohexylring bilden,

A für geradkettiges oder verzweigtes Alkyl oder Alkenyl mit jeweils bis zu 4 Kohlenstoffatomen steht, die gegebenenfalls durch Hydroxy, Phenyl, Cyclobutyl, Cyclohexyl oder durch eine Gruppe der Formel $-NR^{18}R^{19}$, $-NHCO-OR^{20}$, $-SiR^{21}R^{22}R^{23}$ oder $-SnR^{21}R^{22}R^{23}$ substituiert sind,

worin

$R^{18}$ und $R^{19}$ gleich oder verschieden sind und die oben angegebene Bedeutung von $R^4$ haben und mit dieser gleich oder verschieden sind,

$R^{20}$ Methyl oder Ethyl bedeutet, die gegebenenfalls durch Phenyl substituiert sind,

$R^{21}$, $R^{22}$ und $R^{23}$ geradkettiges oder verzweigtes Alkyl mit bis zu 4 Kohlenstoffatomen bedeuten,

oder

für geradkettiges oder verzweigtes Acyl mit bis zu 4 Kohlenstoffatomen steht,
für Cyclobutyl oder Cyclohexyl steht, die gegebenenfalls durch Hydroxy substituiert sind,
für Carboxy, Alkoxycarbonyl mit bis zu 3 Kohlenstoffatomen oder für eine Gruppe der Formel $-SiR^{21}R^{22}R^{23}$, $-SnR^{21}R^{22}R^{23}$ oder $R^{24}$-CO- steht,
worin

$R^{21}$, $R^{22}$ und $R^{23}$ die oben angegebene Bedeutung haben
und

$R^{24}$ Wasserstoff oder geradkettiges oder verzweigtes Alkyl oder Alkenyl mit jeweils bis zu 4 Kohlenstoffatomen bedeutet, die gegebenenfalls durch Phenyl oder durch die Gruppe der Formel $-NH-CO-OR^{20}$ substituiert sind,
worin

$R^{20}$ die oben angegebene Bedeutung hat.

**4.** Verfahren zur Herstellung von Verbindungen der allgemeinen Formel (I)

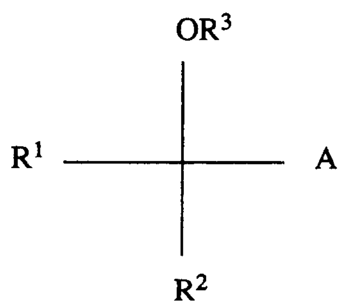
(I),

in welcher

$R^2$ für einen 3- bis 8-gliedrigen, gesättigten oder ungesättigten Heterocyclus mit bis zu 2 Heteroatomen aus der Reihe S oder O oder mit einer Gruppe der Formel$-NR^4$, $-SiR^5R^6R^7$ oder $SnR^5R^6R^7$ steht,
worin

$R^4$ Wasserstoff, geradkettiges oder verzweigtes Alkyl mit bis zu 8 Kohlenstoffatomen, Phenyl oder Benzyl bedeutet,

$R^5$, $R^6$ und $R^7$ gleich oder verschieden sind und geradkettiges oder verzweigtes Alkyl mit bis zu 8 Kohlenstoffatomen oder Phenyl bedeuten,
wobei dieser gegebenenfalls bis zu 3-fach gleich oder verschieden durch geradkettiges oder verzweigtes Alkyl oder Alkoxy mit jeweils bis zu 8 Kohlenstoffatomen, Hydroxy, Phenoxy, Benzyl oder durch einen 5- bis 7-gliedrigen, gesättigten oder ungesättigten Heterocyclus mit bis zu 3 Heteroatomen aus der Reihe S, N oder O, durch Aryl mit 6 bis 10 Kohlenstoffatomen oder durch eine Gruppe der Formel $-NR^8R^9$ substituiert sein kann,
worin

$R^8$ und $R^9$ gleich oder verschieden sind und die oben angegebene Bedeutung von $R^4$ haben,

$R^1$ für Wasserstoff oder für die Gruppe $-SiR^5R^6R^7$ steht,
worin

$R^5$, $R^6$ und $R^7$ die oben angegebene Bedeutung haben,

$R^3$ für eine Gruppe der Formel $-CO-NR^{10}R^{11}$ steht,
worin

$R^{10}$ und $R^{11}$ gemeinsam mit dem Stickstoffatom einen heterocyclischen Rest der Formel

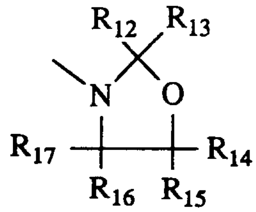

bilden,
worin

| | |
|---|---|
| $R^{12}$, $R^{13}$, $R^{14}$, $R^{15}$, $R^{16}$ und $R^{17}$ | gleich oder verschieden sind und Wasserstoff, geradkettiges oder verzweigtes Alkyl mit bis zu 8 Kohlenstoffatomen, Phenyl oder Cycloalkyl mit 3 bis 6 Kohlenstoffatomen bedeuten oder jeweils $R^{12}$ und $R^{13}$, $R^{14}$ und $R^{15}$ und/oder $R^{16}$ und $R^{17}$ gemeinsam einen 3- bis 6-gliedrigen, gesättigten Carbocyclus bilden, |
| A | für geradkettiges oder verzweigtes Alkyl oder Alkenyl mit jeweils bis zu 8 Kohlenstoffatomen steht, die gegebenenfalls bis zu 3-fach gleich oder verschieden durch Hydroxy, Phenyl oder Cycloalkyl mit 3 bis 7 Kohlenstoffatomen substituiert sind oder durch eine Gruppe der Formel $-NR^{18}R^{19}$, $-HN-CO-OR^{20}$, $-SiR^{21}R^{22}R^{23}$ oder $SnR^{21}R^{22}R^{23}$ substituiert sind,<br>worin |
| $R^{18}$ und $R^{19}$ | die oben angegebene Bedeutung von $R^4$ haben und mit dieser gleich oder verschieden sind<br>und |
| $R^{20}$ | geradkettiges oder verzweigtes Alkyl mit bis zu 6 Kohlenstoffatomen bedeutet, das gegebenenfalls durch Phenyl substituiert ist, |
| $R^{21}$, $R^{22}$ und $R^{23}$ | die oben angegebene Bedeutung von $R^5$, $R^6$ und $R^7$ haben und mit dieser gleich oder verschieden sind,<br>oder<br>für geradkettiges oder verzweigtes Acyl mit bis zu 8 Kohlenstoffatomen steht,<br>für Cycloalkyl oder Cycloalkenyl mit 3 bis 7 Kohlenstoffatomen steht, das gegebenenfalls durch Hydroxy substituiert ist, oder<br>für Carboxy, Alkoxycarbonyl mit bis zu 4 Kohlenstoffatomen oder für eine Gruppe der Formel $-SiR^{21}R^{22}R^{23}$, $-SnR^{21}R^{22}R^{23}$ oder $R^{24}$-CO steht,<br>worin |
| $R^{21}$, $R^{22}$ und $R^{23}$ | die oben angegebene Bedeutung haben<br>und |
| $R^{24}$ | Wasserstoff oder geradkettiges oder verzweigtes Alkyl oder Alkenyl mit jeweils bis zu 8 Kohlenstoffatomen bedeutet, die gegebenenfalls bis zu 3-fach durch Phenyl oder durch die Gruppe der Formel $-NH-CO-OR^{20}$ substituiert sind,<br>worin |
| $R^{20}$ | die oben angegebene Bedeutung hat, |

dadurch gekennzeichnet, daß man Carbamate der allgemeinen Formel (II)

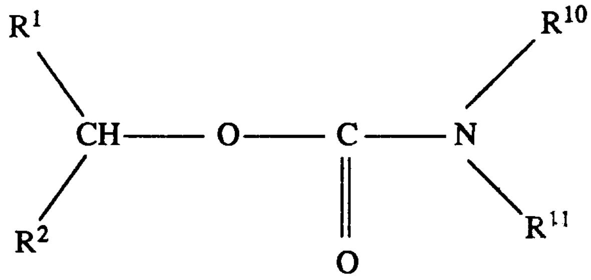

(II),

in welcher

$R^1$, $R^2$, $R^{10}$ und $R^{11}$ die oben angegebene Bedeutung haben,

zunächst in inerten Lösemitteln, in Anwesenheit einer selektiven Base und eines chelatbildenden Diamins (im folgenden mit D bezeichnet) zu den Carbanion-Komplexverbindungen der allgemeinen Formel (III)

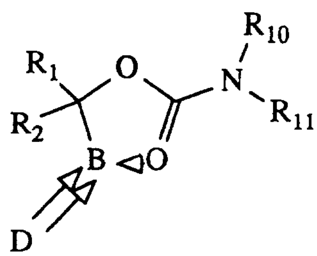

(III),

in welcher

$R^1$, $R^2$, $R^{10}$ und $R^{11}$ die oben angegebene Bedeutung haben,

B für ein Lithium-, Magnesium-, Titan-, Zirkonium- oder Kaliumatom steht,

und

D für ein chelatbildendes Diamin wie beispielsweise Tetramethylethylendiamin (TMEDA) oder (-)-Spartein steht,

enantioselektiv deprotoniert und anschließend mit Elektrophilen der allgemeinen Formeln (IV) und (V)

A-M (IV)

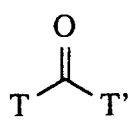

(V),

in welcher

A die oben angegebene Bedeutung hat,

M für Halogen, für $C_1$-$C_4$-Alkoxy oder für eine andere typische Abgangsgruppe steht,

und

T und T' gleich oder verschieden sind und für einen unter dem Substituenten A aufgeführten chemisch sinnvollen Rest stehen,

oder mit $CO_2$ umsetzt.

**5.** Carbamate der allgemeinen Formel (II)

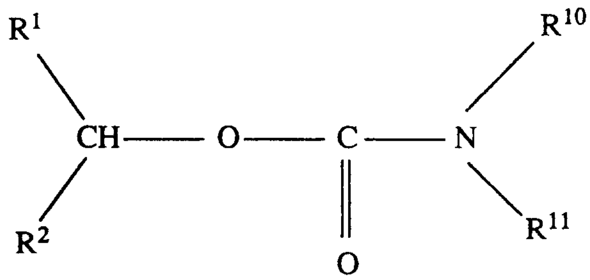 (II),

in welcher
$R^1$, $R^2$, $R^{10}$ und $R^{11}$ die in Anspruch 4 angegebene Bedeutung haben.

**6.** Verfahren zur Herstellung von Carbamaten der allgemeinen Formel (II) gemäß Anspruch 4, dadurch gekennzeichnet, daß man Verbindungen der allgemeinen Formel (VI)

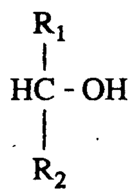 (VI),

in welcher
$R^1$ und $R^2$ die in Anspruch 4 angegebene Bedeutung haben,
mit Verbindungen der allgemeinen Formel (VII)

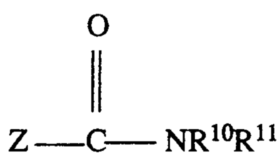 (VII),

in welcher
$R^{10}$ und $R^{11}$ die in Anspruch 4 angegebene Bedeutung haben
und
Z für Halogen steht,
in einem inerten Lösungsmittel, in Anwesenheit einer Base, bei Temperaturen zwischen 0 °C und +130 °C umsetzt.

**7.** Verwendung der Verbindungen der allgemeinen Formel (I) gemäß Anspruch 1 bei der Herstellung von 1-Hydroxy-substituierten Cycloalkylverbindungen die für die Synthese von biologisch aktiven Peptiden und Peptid-Mimetika eingesetzt werden können.